# EUROPEAN PATENT APPLICATION

(11) **EP 4 692 086 A1**
(43) Date of publication of application: **11.02.2026**
(21) Application number: 24777885.5
(22) Date of filing: 22.03.2024
(51) Int. Cl.: C07D 473/04, A61K 31/52, A61P 7/00

(54) **ANTICOAGULATION REVERSAL AGENT, AND PREPARATION METHOD THEREFOR AND USE THEREOF**

(30) Priority: 24.03.2023 CN 202310305256
(71) Applicant: Shaanxi Micot Pharmaceutical Technology Co., Ltd., Xi 'an, Shaanxi 710116 (CN)
(72) Inventor: ZHENG, Du, Xi'an, Shaanxi 710116 (CN); LIU, Xingxin, Xi'an, Shaanxi 710116 (CN)
(74) Representative: ABG Intellectual Property Law, S.L.
(86) International application number: PCT/CN2024/083158
(87) International publication number: WO 2024/199096

(57) **Abstract**

Provided in the present invention is an anticoagulation reversal agent having a structure as shown in formula I or a salt thereof or a deuterated compound thereof. Experimental results show that the compound provided in the present invention can better inhibit bleeding complications caused by an anticoagulant, for example, the compound can inhibit bleeding complications caused by warfarin, heparin and enoxaparin sodium, and has the characteristics of broad spectrum and high efficiency.

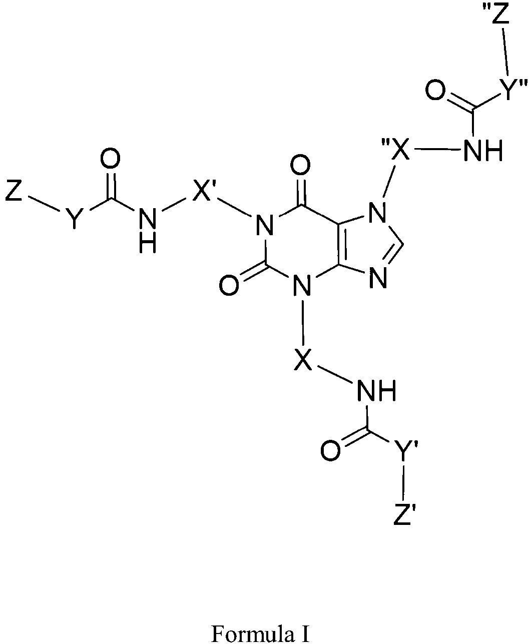

## Description

This application claims the priority of Chinese Patent Application No. 202310305256.2, filed with the China National Intellectual Property Administration on March 24, 2023, and titled with "ANTICOAGULATION REVERSAL AGENT, AND PREPARATION METHOD THEREFOR AND USE THEREOF", which is hereby incorporated by reference in its entirety.

### FIELD

The present disclosure relates to the field of medicinal chemistry technology, in particular to an anticoagulation reversal agent, and preparation method therefor and use thereof.

### BACKGROUND

Currently, there are approximately 423 million patients with cardiovascular diseases, in which a considerable number of patients require treatment with anticoagulant drugs. Common anticoagulant drugs include: 1. vitamin K antagonists, which achieve an anticoagulation effect by antagonizing vitamin K to reduce the synthesis of prothrombin and coagulation factors VIIIX and X in liver, as represented by drugs such as warfarin; 2. indirect thrombin inhibitors which achieve an anticoagulation effect by interacting with antithrombin, thereby indirectly inhibiting the activity of factors Xa and IIa, as represented by drugs such as heparin and low molecular weight heparin; 3. direct thrombin inhibitors which block the last step of the coagulation cascade and thrombus formation by inhibiting thrombin and thereby inhibiting the cleavage of fibrinogen into fibrin, wherein monovalent thrombin inhibitors (such as dabigatran etexilate, argatroban) can directly inhibit thrombin, and divalent thrombin inhibitors (such as bivalirudin, recombinant hirudin) can directly inhibit thrombin while separating thrombin and fibrin to achieve anticoagulation effect.

However, anticoagulant therapy is a double-edged sword. On one hand, it is a fundamental therapy for preventing and treating thrombotic diseases, and can significantly reduce the incidence of thrombotic events. On the other hand, it can lead to bleeding complications, which can be life-threatening in severe cases. Therefore, there is a clinical need for an antagonist that can rapidly and effectively reverse the effects of anticoagulation to provide a greater safety margin for a large amount of patients undergoing anticoagulant therapy. At present, there are relatively few anticoagulation reversal agents available on the market or under development. Marketed agents include vitamin K as a reversal agent for warfarin, protamine for heparin, monoclonal antibody Idarucizumab for dabigatran etexilate, and anticoagulation reversal agent Andexxa for factor Xa inhibitors Rivaroxaban/Apixaban. Patent publication WO2013082210A1 has disclosed an anticoagulation reversal agent diarginine piperazine (DAP, PER977), which can reverse the anticoagulation effect of heparin, heparin fragments, fondaparinux, and inhibitors of factors Xa or IIa (such as oral inhibitors of factor Xa or IIa), and is currently in phase III clinical trials.

At present, there is a lack of broad-spectrum and high-effective anticoagulation reversal agents in clinical practice.

### SUMMARY

In view of the foregoing, a technical problem to be solved in the present disclosure is to provide an anticoagulation reversal agent, a preparation method therefor, and a use thereof, wherein the prepared anticoagulation reversal agent features a broad spectrum and a high efficiency.

The present disclosure provides a compound having a structure as represented by formula I: 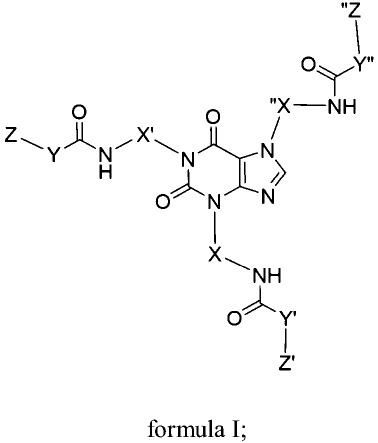
wherein, X, X', and X" are independently selected from the group consisting of substituted or unsubstituted alkyl, alkenyl, and heterocyclyl;
Y, Y', and Y" are independently selected from the group consisting of substituted or unsubstituted alkyl, alkenyl, and heterocyclyl; and
Z, Z', and Z" are independently selected from the group consisting of heteroatom-containing molecular fragments which can be protonated under physiological conditions;
or a salt thereof or a deuterated form thereof.

Preferably, the alkyl has 1 to 10 carbon atoms, more preferably 1 to 6 carbon atoms, and further preferably 2, 3, 4, or 5 carbon atoms.

Preferably, the alkenyl has 2 to 10 carbon atoms, more preferably 2 to 6 carbon atoms, and further preferably 2, 3, 4, or 5 carbon atoms.

Preferably, the heterocyclyl has 2 to 12 carbon atoms; more preferably, the heterocyclyl is monocyclic; further preferably, the heterocyclyl is a five- or six-membered heterocyclyl. Preferably, heteroatom in the heterocyclyl is one or more selected from the group consisting of N, O, and S.

Preferably, a substituent on the alkyl, alkenyl, or heterocyclyl is one or more independently selected from the group consisting of an amino group, a nitro group, and a halogen; more preferably, the substituent is an amino group.

Preferably, the Y-Z, Y'-Z', Y"-Z" are independently selected from the group consisting of residues of basic amino acids; more preferably, the residues of basic amino acids are selected from the group consisting of a residue of histidine, a residue of arginine, and a residue of lysine.

In some specific embodiments, the residue refers to a group remaining after a carboxyl group is removed from an amino acid.

Preferably, the heteroatom-containing molecular fragments are selected from the group consisting of an amino group, a guanidino group, and an imidazolyl group.

Preferably, the compound has a structure as represented by formula I-a: 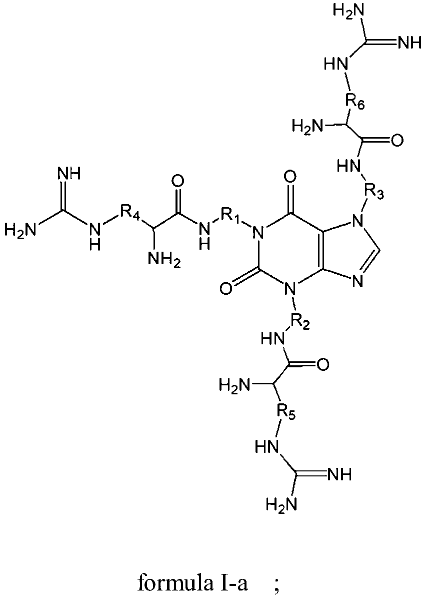 wherein R₁, R₂, R₃, R₄, R₅, and R₆ are independently selected from the group consisting of substituted or unsubstituted C₁-C₆ alkyl, C₂-C₆ alkenyl, and monocyclic heterocyclyl; more preferably C₂-C₅ alkyl, C₂-C₅ alkenyl, and monocyclic heterocyclyl; further preferably a methylene group, an ethylene group, an n-propylene group, an isopropylene group, an n-butylene group, an isobutylene group, a tert-butylene group, an n-pentylene group, an isopentylene group, a vinylene group, a propenylene group, a butenylene group, a pyrrolylene group, a pyridinylene group, a thiophenylene group, and a furanylene group.

Preferably, the compound has a structure of:

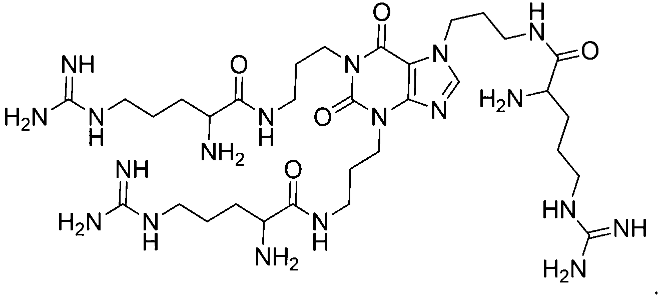

Preferably, the compound has a structure of:

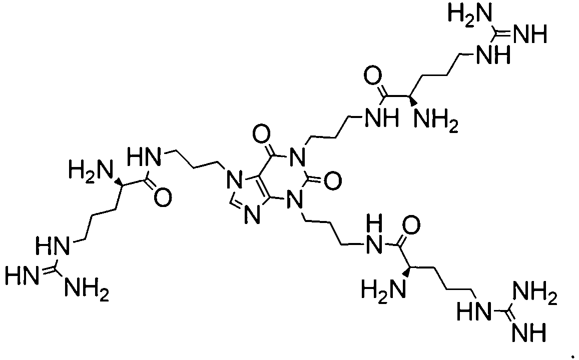

The present disclosure provides a method for preparing the aforementioned compounds , comprising steps of:
S1) performing reaction with xanthine and a halide as represented by formula a, to obtain an intermediate 1;
S2) subjecting the intermediate 1 to an amino deprotection treatment;
S3) performing reaction with the deprotected compound and compound as represented by formula b, to obtain a compound 2; and
S4) subjecting the compound 2 to a deprotection treatment, to obtain a compound as represented by formula I;

   BocHN-X-Br formula a;

   Z-Y-COOH formula b;
wherein, X is selected from the group consisting of substituted or unsubstituted alkyl, alkenyl, and heterocyclyl;
Y is selected from the group consisting of substituted or unsubstituted alkyl, alkenyl, and heterocyclyl; and
Z is selected from the group consisting of heteroatom-containing molecular fragments that can be protonated under physiological conditions.

The present disclosure provides an anticoagulation reversal composition, comprising a compound mentioned above and a pharmaceutically acceptable excipient.

The types of the aforementioned excipient are not particularly limited and can be any suitable excipients known to those skilled in the art.

The present disclosure provides use of a compound mentioned above or the aforementioned anticoagulation reversal composition in the manufacture of a medicament for preventing, treating or ameliorating hemorrhage caused by an anticoagulant.

Preferably, the anticoagulant is one or more selected from the group consisting of vitamin K antagonists and indirect thrombin inhibitors.

More preferably, the vitamin K antagonist is warfarin, and the indirect thrombin inhibitor is heparin or low molecular weight heparin. The aforementioned anticoagulation reversal agent can also be used in combination with other anticoagulation reversal agents.

The present disclosure also provides a kit comprising a compound mentioned above and one or more anticoagulants.

Preferably, the anticoagulants are one or more selected from the group consisting of vitamin K antagonists and indirect thrombin inhibitors.

More preferably, the vitamin K antagonist is warfarin, and the indirect thrombin inhibitor is heparin or low molecular weight heparin.

Compared to the prior art, the present disclosure provides a compound having a structure as represented by formula I or a salt thereof or a deuterated form thereof. Experimental results showed that the compound provided in the present disclosure can effectively inhibit bleeding complications caused by an anticoagulant, for example, the compound can inhibit bleeding complications caused by warfarin, heparin and enoxaparin sodium, and features a broad spectrum and a high efficiency.

### BRIEF DESCRIPTION OF DRAWINGS

FIG. 1 is a mass spectrum of compound 1;
FIG.2 is a ¹H nuclear magnetic resonance spectrum of compound 1.

### DETAILED DESCRIPTION

To further illustrate the present disclosure, the anticoagulation reversal agent and the preparation method therefor and use thereof, provided by the present disclosure, are described in detail below with reference to the following examples.

### Example 1

### First step:

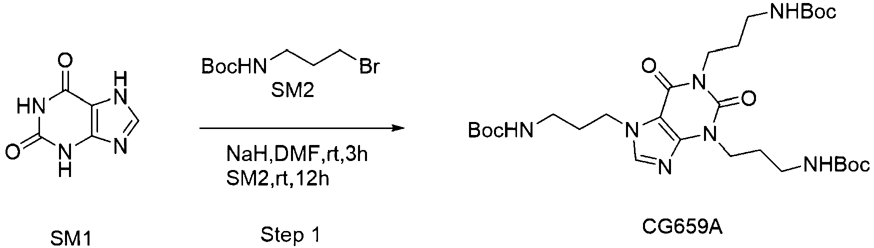

### Operation procedure

1. to a 1000 mL three-necked flask, DMF (500 mL) was added, and stirring was initiated;
2. the temperature was maintained at 0 to 10 °C, and NaH (27.6 g) was added slowly;
3. the temperature was maintained at 0 to 10 °C, and xanthine (21 g) was added slowly;
4. the mixture was warmed to room temperature and stirred for 1 h;
5. N-Boc-bromopropionamine (115 g) was added slowly;
6. the mixture was stirred at room temperature overnight, and then the stirring was stopped;

### Post-processing

7. the reaction solution was added into 1000 mL of ice water, and then extracted by 1000 mL of dichloromethane (twice, 1000 mL*2);
8. the organic phase was washed with saturated brine twice (500 mL*2);
9. the organic phase was concentrated to dryness;
10. the residue of the concentration was purified using silica-gel column chromatography (eluent: ethyl acetate/methyl tert-butyl ether=1/1)
11. product-containing fractions were collected and concentrated to dryness;
12. a colorless oily substance was obtained.

### Second step:

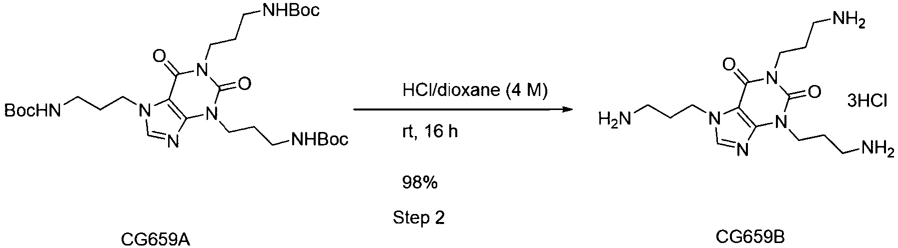

### Operation procedure

1. to a flask containing oily substance CG659A, dioxane was added and the mixture was stirred until dissolved;
2. the temperature was maintained at 10±5°C, and a solution of dioxane in hydrochloric acid was added dropwise and slowly;
3. the temperature was maintained at 20±5°C, and the mixture was stirred for 16 h;
4. a solid product precipitated;

### Post-processing

5. a resulting mixture was filtered under reduced pressure with nitrogen protection, and a resulting filter cake was washed with dioxane (25 mL*2);
6. the filter cake was added into methyl tert-butyl ether (150 mL), and the mixture were stirred at room temperature for 1 h;
7. the mixture was filtered under reduced pressure with nitrogen protection, and a resulting filter cake was washed with methyl tert-butyl ether (25 mL).

### Third step:

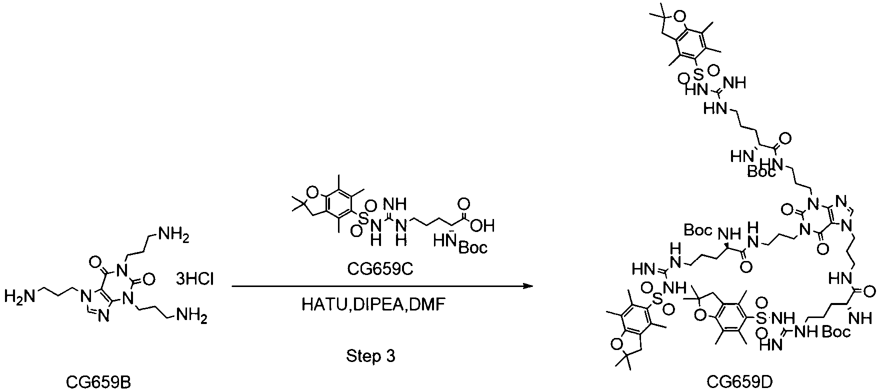

### Operation procedure

1. to a 100 mL three-necked flask, Boc-L-Arg(Pbf)-OH (CG659C, 2.0 g, 3.3 eq) and DMF (30 mL) were added, and then DIPEA (1.5 g, 10 eq) was added under stirring;
2. the resulting mixture was cooled to 0±5°C, then HATU (1.5 g, 3.3 eq) was added, and the temperature was maintained for 30 min;
3. the temperature was maintained at 0±5°C, and CG659-B (0.5 g, 1.0eq) was added;
4. the mixture was warmed to room temperature, and the reaction was allowed to proceed overnight (16 h);
5. water (30 mL) was added to the resulting reaction solution, and a solid precipitated and was filtered under reduced pressure;
6. the solid was dissolved in ethyl acetate, washed with water, and concentrated to dryness, to obtain 1.9 g of thick substance;
7. the resulting filtrate was extracted by methyl tert-butyl ether (2*30 mL);
8. the resulting organic phases were combined and washed with water (25 mL);
9. the organic phase was concentrated to dryness to obtain 0.3 g of oily substance.

### Fourth step:

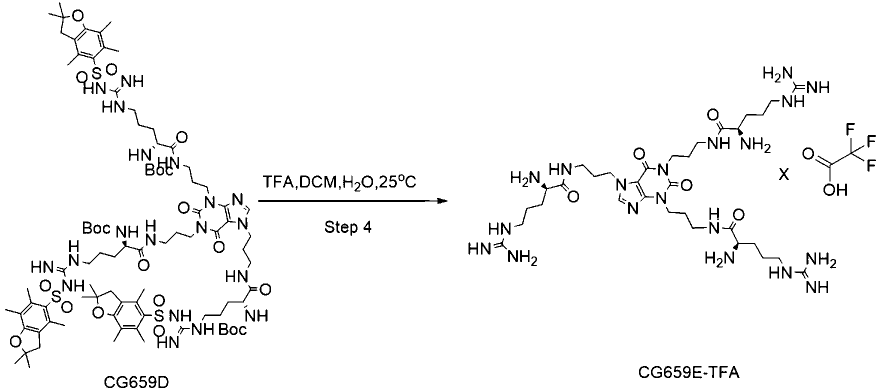

### Operation procedure

1. to a 100 mL three-necked reaction flask, a solution of CG659D/DCM (0.5 g/3 mL) was added;
2. TFA (1 mL, 50 eq), water (29.2 mg, 6.0 eq), and TIS (141 mg, 3.3 eq) were added at room temperature;
3. the mixture was stirred at room temperature overnight, TFA (2 mL) was supplementally added, and stirring was continued for 5 h.

### Post-processing

4. DCM and a majority of TFA in the system were removed by concentration, to obtain 0.7 g of crude product;
5. anhydrous ethanol (10 mL) was added under stirring, a solid precipitated, and the mixture was stirred for 1 h;
6. the resulting mixture was filtered under reduced pressure, the resulting filter cake was washed with MTBE (2×2 mL) by dripping, and 0.3 g of off-white solid product was obtained; 3 mL of water was added and pH was adjusted to 8 to 9 with 5% NaOH aqueous solution; the resulting mixture was extracted with DCM (2×10 mL) and 0.06 g oily substance was obtained; the substance was azeotropically dried with 100 mL toluene in batches, dissolved by adding 50 mL isopropanol, and filtered; the resulting filtrate was concentrated by rotary evaporation to obtain about 0.2 g of oily liquid. The product was denoted as compound 1.

### Structural characterization:

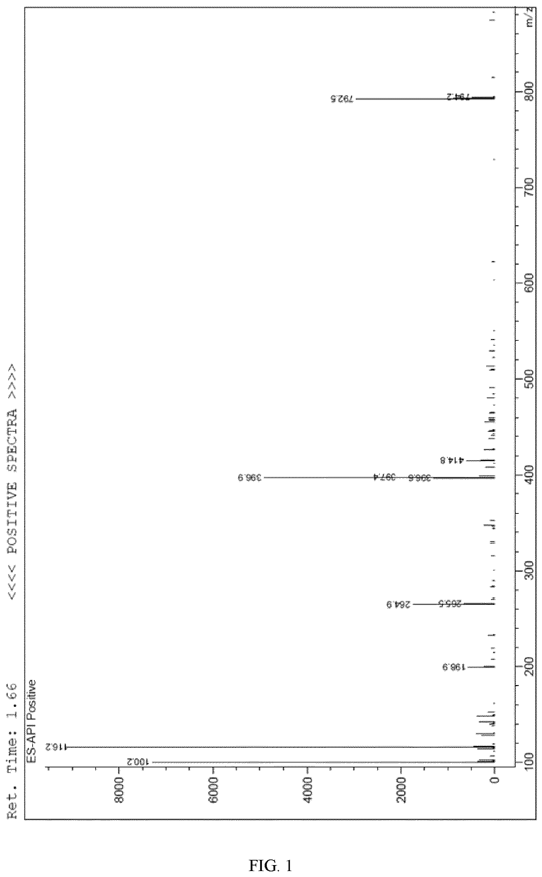

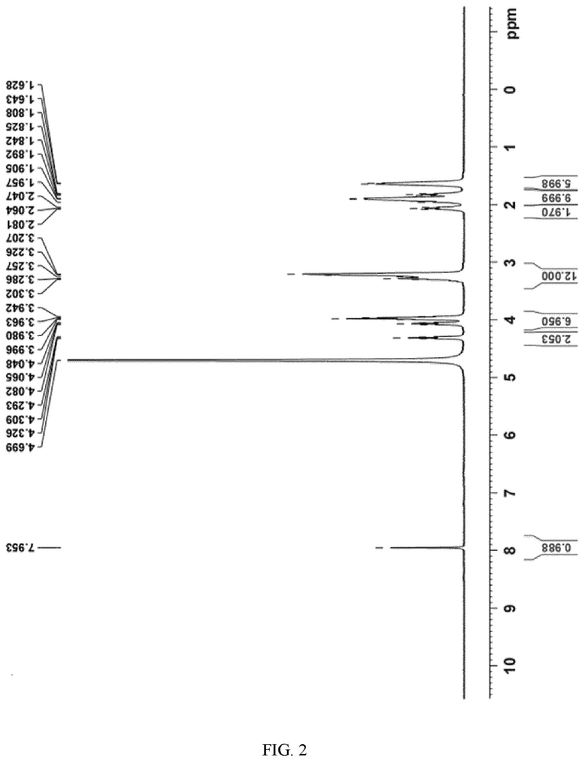

Structural characterization was performed via mass spectrum and nuclear magnetic resonance, and the results were shown in FIG. 1 and 2.

Mass spectrometry result: [M+H]⁺: 792.5.

The product had a free base molecular weight of 791.5 according to the molecular ion peaks obtained in the low-resolution mass spectrometry in FIG. 1. Furthermore, according to the nitrogen rule, the product had an exact molecular weight of an odd number, which was in line with the chemical formula of the target product.

The structure of the product was confirmed by results of nuclear magnetic resonance.

### Example 2: antagonizing warfarin

### Experimental procedure

Tail transection hemorrhage model: In all groups except for Control group and Compound 1 20 mg/kg+N.S. control group, SD rats were administered warfarin by oral gavage once daily for 3 consecutive days. 30 min after the final administration, the rats were injected with 10% chloral hydrate via intraperitoneal injection. The anaesthetized animals were placed on an electric heating blanket preheated to 37°C. Jugular vein was isolated and cannulated within 30 min. Normal saline or drug was injected via the jugular vein. 5 min after injection, a transection was made 5 mm away from the tip of the rat's tail. Bleeding loss (BL) over 15 min was recorded. At the end of the experiment, blood was collected from the abdominal cavity and WBCT was measured. Temperatures including room temperature and water bath temperature were strictly controlled throughout the experiment.

Hepatic laceration hemorrhage model: In all groups except for Control group and Compound 1 20 mg/kg+N.S. control group, SD rats were administered warfarin by oral gavage once daily for 3 consecutive days. 30 min after the final administration, the rats were injected with 10% chloral hydrate via intraperitoneal injection. The anaesthetized animals were placed on an electric heating blanket preheated to 37°C. Jugular vein was isolated and cannulated within 30 min. Aspirin was administered and 30 min later, test substances were injected via the jugular vein. 5 min after injection, the abdominal cavity was opened, and 3 standard incisions, each with a length of 1 cm and a depth of 2 mm, were made on the liver. Blood flowing from the liver was absorbed with pre-weighed dry cotton, and bleeding loss (BL) over 30 min was recorded.

The results of the experiment are shown in Table 1 to 3.

**Table 1: Effect on hepatic laceration bleeding loss in warfarin-induced rats**

| Group | n | Hepatic Laceration Bleeding Loss (g) | Inhibition Rate |
|---|---|---|---|
| Control (N.S.) | 3 | 0.0431±0.0152 | - |
| Compound 1 Control (Compound 1 20 mg/kg+N.S.) | 3 | 0.0561±0.0028 | - |
| Vehicle (warfarin) | 3 | 0.2808±0.1563 | - |
| PER977 20mg/kg (PER977 20mg/kg+warfarin) | 3 | 0.0874±0.0292 | 68.9 |
| Compound 1 5 mg/kg (Compound 1 5 mg/kg+warfarin) | 3 | 0.3029±0.1189 | -7.9 |
| Compound 1 10 mg/kg (Compound 1 10 mg/kg+warfarin) | 3 | 0.2379±0.1613 | 15.3 |
| Compound 1 20 mg/kg (Compound 1 20 mg/kg+warfarin) | 3 | 0.0784±0.0346 | 72.1 |

**Table 2: Effect on tail transection bleeding loss in warfarin-induced rats**

| Group | n | Tail Transection Bleeding Loss (g) |
|---|---|---|
| Control (N.S.) | 10 | 0.0098±0.0038 |
| Vehicle (warfarin) | 10 | 0.1076±0.0768 |
| PER977 20mg/kg (PER977 20mg/kg+warfarin) | 10 | 0.0276±0.0123 |
| Compound 1 20 mg/kg (Compound 1 20 mg/kg+warfarin) | 9 | 0.0143±0.0108 |

**Table 3: Effect on WCBT in warfarin-induced rats**

| Group | n | WBCT (s) |
|---|---|---|
| Control (N.S.) | 10 | 357±41 |
| Vehicle (warfarin) | 10 | 687±130 |
| PER977 20mg/kg (PER977 20mg/kg+warfarin) | 10 | 471±101 |
| Compound 1 20 mg/kg (Compound 1 20 mg/kg+warfarin) | 9 | 490±99 |

The results showed that, compared to the Control group, tail transection bleeding loss in rats of the Vehicle group was significantly increased (P<0.05), hepatic laceration bleeding loss was also significantly increased, and WBCT was significantly prolonged as well (P<0.001). Compared to the Vehicle group, 20 mg/kg compound 1 significantly reduced the tail transection bleeding loss in rats (P<0.05), showing superiority to PER977; 20 mg/kg compound 1 inhibited hepatic laceration bleeding loss in rats by 72.1%, and also significantly shortened WBCT (P<0.001). Compared to the Control group, Compound 1 control group (Compound 1 20 mg/kg+N.S.) showed no significant changes on bleeding loss or WBCT.

### Example 3: antagonizing heparin

### Experimental procedure

Tail transection hemorrhage model: In all groups except for Control group and Compound 1 20 mg/kg+N.S. control group, SD rats were administered heparin by tail vein injection, followed immediately by injection with 10% chloral hydrate via intraperitoneal injection. The anaesthetized animals were placed on an electric heating blanket preheated to 37°C. Jugular vein was isolated and cannulated within 30 min. Normal saline or drug was injected via the jugular vein. 5 min after injection, a transection was made 5 mm away from the tip of the rat's tail. Bleeding loss (BL) over 15 min was recorded. Temperatures including room temperature and water bath temperature were strictly controlled throughout the experiment.

Hepatic laceration hemorrhage model: In all groups except for Control group and Compound 1 20 mg/kg+N.S. control group, SD rats were administered heparin by tail vein injection, followed immediately by injection with 10% chloral hydrate via intraperitoneal injection. The anaesthetized animals were placed on an electric heating blanket preheated to 37°C. Jugular vein was isolated and cannulated within 30 min. Aspirin was administered and 30 min later, test drugs were injected via the jugular vein. 5 min after injection, the abdominal cavity was opened, and 3 standard incisions, each with a length of 1 cm and a depth of 2 mm, were made on the liver. Blood flowing from the liver was absorbed with pre-weighed dry cotton, and bleeding loss (BL) over 30 min was recorded.

The results of the experiment are shown in Table 4.

**Table 4: Effect on bleeding loss in heparin-induced rats**

| Group | n | Tail Transection Bleeding Loss (mg) | Hepatic Laceration Bleeding Loss (g) |
|---|---|---|---|
| Control (N.S.) | 8 | 10.0875±8.9912 | 0.0774±0.0367 |
| Compound 1 Control (Compound 1 20 mg/kg+N.S.) | 8 | 6.3250±2.9295 | 0.0326±0.0267 |
| Vehicle (heparin) | 8 | 62.2875±19.6503 | 0.5035±0.2793 |
| PER977 20mg/kg (PER977 20mg/kg+heparin) | 8 | 17.9000±11.6004 | 0.0511±0.0444 |
| Compound 1 5 mg/kg (Compound 1 5 mg/kg+heparin) | 8 | 38.3625±12.6991 | 0.3217±0.0919 |
| Compound 1 10 mg/kg (Compound 1 10 mg/kg+heparin) | 8 | 19.9375±22.3215 | 0.1794+0.0966 |
| Compound 1 20 mg/kg (Compound 1 20 mg/kg+heparin) | 8 | 12.1125±6.6714 | 0.0560+0.0408 |

The experimental results showed that, compared to the Control group, tail transection bleeding loss and hepatic laceration bleeding loss in rats of the Vehicle group was significantly increased (P<0.05, P<0.001), wherein the hepatic laceration bleeding loss increased by 5.5 times. Compared to the Vehicle group, 10 mg/kg compound 1 significantly reduced the tail transection bleeding loss and hepatic laceration bleeding loss in rats (P<0.05, P<0.01), wherein the inhibition rate for hepatic laceration bleeding loss in rats reached 88.9%, showing superiority to PER977 under equal dose. Compared to the Control group, Compound 1 control group (Compound 1 20 mg/kg+N.S.) showed no significant change on bleeding loss.

### Example 4: antagonizing enoxaparin

### Experimental procedure

Tail transection hemorrhage model: In all groups except for Control group and Compound 1 5.0 mg/kg+N.S. control group, SD rats were administered enoxaparin by tail vein injection, followed immediately by injection with 10% chloral hydrate via intraperitoneal injection. The anaesthetized animals were placed on an electric heating blanket preheated to 37°C. Jugular vein was isolated and cannulated within 30 min. Normal saline or drug was injected via the jugular vein. 5 min after injection, a transection was made 5 mm away from the tip of the rat's tail. Bleeding loss (BL) over 15 min was recorded. Temperatures including room temperature and water bath temperature were strictly controlled throughout the experiment.

Hepatic laceration hemorrhage model: In all groups except for Control group and Compound 1 5.0 mg/kg+N.S. control group, SD rats were administered enoxaparin by tail vein injection, followed immediately by injection with 10% chloral hydrate via intraperitoneal injection. The anaesthetized animals were placed on an electric heating blanket preheated to 37°C. Jugular vein was isolated and cannulated within 30 min. Aspirin was administered and 30 min later, test drugs were injected via the jugular vein. 5 min after injection, the abdominal cavity was opened, and 3 standard incisions, each with a length of 1 cm and a depth of 2 mm, were made on the liver. Blood flowing from the liver was absorbed with pre-weighed dry cotton, and bleeding loss (BL) over 30 min was recorded.

The results of the experiment were shown in Table 5 to 6.

**Table 5: Effect on tail transection bleeding loss in enoxaparin-induced rats**

| Group | n | Tail Transection Bleeding Loss (g) |
|---|---|---|
| Control (N.S.) | 8 | 0.0164+0.0104 |
| Compound 1 Control (Compound 1 5 mg/kg+N.S.) | 8 | 0.0136±0.0087 |
| Vehicle (enoxaparin) | 8 | 0.0944±0.0667 |
| PER977 20mg/kg (PER977 20mg/kg+enoxaparin) | 8 | 0.0228±0.0155 |
| Compound 1 0.2 mg/kg (Compound 1 0.2 mg/kg+enoxaparin) | 8 | 0.0903±0.0426 |
| Compound 1 1 mg/kg (Compound 1 1 mg/kg+enoxaparin) | 8 | 0.0302±0.0147 |
| Compound 1 5 mg/kg (Compound 1 5 mg/kg+enoxaparin) | 8 | 0.0158±0.0083 |

**Table 6: Effect on hepatic laceration bleeding loss in enoxaparin-induced rats**

| Group | n | Hepatic Laceration Bleeding Loss (g) | Inhibition Rate (%) |
|---|---|---|---|
| Control (N.S.) | 10 | 0.0558±0.0236 | |
| Compound 1 Control (Compound 1 5 mg/kg+N.S.) | 10 | 0.0466±0.0246 | |
| Vehicle (enoxaparin) | 10 | 0.4485±0.1068 | |
| PER977 20mg/kg (PER977 20mg/kg+enoxaparin) | 10 | 0.0420±0.0244 | 90.6 |
| Compound 1 0.2 mg/kg (Compound 1 0.2 mg/kg+enoxaparin) | 10 | 0.5253±0.2034 | -17.1 |
| Compound 1 1 mg/kg (Compound 1 1 mg/kg+enoxaparin) | 10 | 0.1674±0.0734 | 62.7 |
| Compound 1 5 mg/kg (Compound 1 5 mg/kg+enoxaparin) | 10 | 0.0490±0.0184 | 89.1 |

The experimental results showed that, compared to the Control group, hepatic laceration bleeding loss in rats of the Vehicle group was significantly increased (P<0.001), wherein the hepatic laceration bleeding loss increased by 7 times. Compared to the Vehicle group, compound 1 significantly reduced the tail transection bleeding loss in rats, wherein the 5 mg/kg group was superior to the 20 mg/kg PER977 group; the 1 mg/kg and 5 mg/kg compound 1 groups significantly reduced the hepatic laceration bleeding loss in rats (P<0.001), wherein the inhibition rate reached 62.7% and 89.1%, respectively, and the 5 mg/kg group had an inhibition rate comparable to that of 20 mg/kg PER977 group. Compared to the Control group, Compound 1 control group (Compound 1 5 mg/kg+N.S.) showed no significant change on bleeding loss.

Experimental results above showed that the compound prepared in the present disclosure exhibited a better anticoagulation reversal effect compared to PER977.

The foregoing experimental examples are provided for illustrative purposes only to facilitate an understanding of the method and core principles of the present disclosure. It should be noted that for those of ordinary skill in the art, various improvements and modifications can be made to the present disclosure without departing from the principles thereof. Such improvements and modifications also fall within the scope of protection of the appended claims.

## Claims

1. A compound having a structure as represented by formula I: 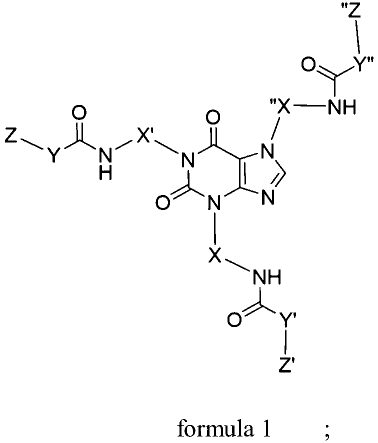
wherein, X, X', and X" are independently selected from the group consisting of substituted or unsubstituted alkyl, alkenyl, and heterocyclyl;
Y, Y', and Y" are independently selected from the group consisting of substituted or unsubstituted alkyl, alkenyl, and heterocyclyl; and
Z, Z', and Z" are independently selected from the group consisting of heteroatom-containing molecular fragments which can be protonated under physiological conditions;
or a salt thereof or a deuterated form thereof.

2. The anticoagulation reversal agent according to claim 1, wherein the alkyl has 1 to 10 carbon atoms, and the alkenyl has 2 to 10 carbon atoms;
and the heterocyclyl has 2 to 12 carbon atoms.

3. The compound according to claim 1, wherein the Y-Z, Y'-Z', Y"-Z" are independently selected from the group consisting of residues of basic amino acids; preferably, the residues of basic amino acids are independently selected from the group consisting of the residues of histidine, arginine, and lysine.

4. The compound according to claim 1, wherein the heteroatom-containing molecular fragments are selected from the group consisting of an amino group, a guanidino group, and an imidazolyl group.

5. The compound according to claim 1, wherein the compound has a structure as represented by formula I-a: 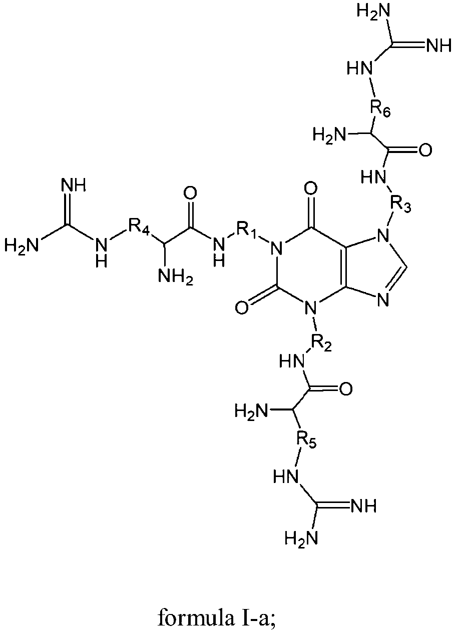 wherein R₁, R₂, R₃, R₄, R₅, and R₆ are independently selected from the group consisting of substituted or unsubstituted C₁-C₆ alkyl, C₂-C₆ alkenyl, and monocyclic heterocyclyl.

6. The compound according to claim 1, wherein the compound has a structure of:

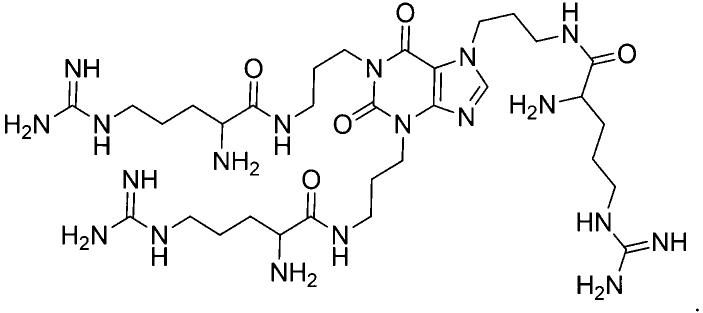

7. The compound according to claim 1, wherein the compound has a structure of:

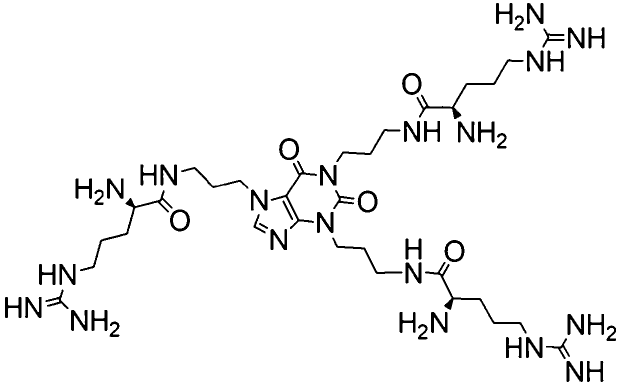

8. A method for preparing the compound according to any one of claims 1 to 7, comprising steps of:
S1) reacting xanthine with a halide as represented by formula a, to obtain an intermediate 1;
S2) subjecting the intermediate 1 to an amino deprotection treatment;
S3) reacting the deprotected compound with a compound as represented by formula b, to obtain a compound 2; and
S4) subjecting the compound 2 to a deprotection treatment, to obtain a compound as represented by formula I;
BocHN-X-Br formula a;
Z-Y-COOH formula b;
wherein, X is selected from the group consisting of substituted or unsubstituted alkyl, alkenyl, and heterocyclyl;
Y is selected from the group consisting of substituted or unsubstituted alkyl, alkenyl, and heterocyclyl; and
Z is selected from the group consisting of heteroatom-containing molecular fragments which can be protonated under physiological conditions.

9. An anticoagulation reversal composition, comprising the compound according to any one of claims 1 to 7, and a pharmaceutically acceptable excipient.

10. Use of the compound according to any one of claims 1 to 7, or the anticoagulation reversal composition according to claim 9, in the manufacture of a medicament for preventing, treating or ameliorating hemorrhage caused by an anticoagulant; preferably, the anticoagulant is one or more selected from the group consisting of vitamin K antagonists and indirect thrombin inhibitors; more preferably, the vitamin K antagonist is warfarin, and the indirect thrombin inhibitor is heparin or low molecular weight heparin.

11. A kit comprising the compound according to any one of claims 1 to 7 and one or more anticoagulants; preferably, the anticoagulant is selected from the group consisting of a vitamin K antagonist and an indirect thrombin inhibitor; more preferably, the vitamin K antagonist is warfarin, and the indirect thrombin inhibitor is heparin or low molecular weight heparin.
